# EUROPEAN PATENT SPECIFICATION

(11) **EP 2 320 870 B1**
(45) Date of publication and mention of the grant of the patent: **09.05.2012**
(21) Application number: 09787835.9
(22) Date of filing: 06.08.2009
(51) Int. Cl.: A61K 9/00

(54) **TOPICAL INTRANASAL COMPOSITION USABLE IN THE CASE OF NASAL OBSTRUCTION**
TOPISCHE INTRANASALE ZUSAMMENSETZUNG ZUR VERWENDUNG BEI NASENVERSTOPFUNGEN
COMPOSITION INTRANASALE TOPIQUE UTILISABLE DANS LE CAS D'UNE OBSTRUCTION NASALE

(30) Priority: 07.08.2008 IT RM20080444
(43) Date of publication of application: 18.05.2011
(73) Proprietor: D.M.G. Italia Srl, 00040 Pomezia (RM) (IT)
(72) Inventor: MERCURI, Luigi, I-00040 Pomezia (RM) (IT)
(74) Representative: Sarpi, Maurizio
(86) International application number: PCT/IT2009/000371
(87) International publication number: WO 2010/016088

(56) References cited:
- WO-A-98/51334
- WO-A-03/051281
- US-A1- 2002 054 917

## Description

The present invention relates to a topical intranasal composition that is able to act against nasal obstruction.

It is known that nasal obstruction may present in different forms, which are at times difficult to identify and of which frequently the attempt is made to diagnose the pathogenetic causes.

In fact, by "respiratory nasal obstruction" is generically meant all those situations that do not allow an adequate flow of air through the nasal airways and that can be caused by alterations of the anatomical structures (nasal septum and turbinates) or by the thickening of the mucosae that coat the surface of the nasal airways. On account of an inflammation, such as for example an allergic rhinitis or a vasomotor rhinitis, an obstruction is created to the passage of air through the nose, which makes it necessary to breathe through the mouth, with consequent snoring and obstructive sleep apnea.

In a healthy nasal fossa, as in a perfect air conditioner, the air drawn in is filtered, humidified, and heated. Filtering takes place, first mechanically, through the hairs and the vibratile cilia of the nasal mucosa, then chemically by the nasal mucus that withholds, incorporating them, the dust and the germs. These, after being subjected to a true bactericidal action performed by lisozyme of the nasal mucus, are pushed towards the pharynx. Heating of the air drawn in is determined by the vascular tissue of the nasal mucosa, automatically ensuring thermal regulation of the nasal cavity. The nasal mucosa normally maintains a state of moisture on its entire surface by means of a clear mucus; this physiological secretion does not appear on the outside (and in fact a healthy nose does not need to be blown) but is subject to attack by bacteria and viruses, the proliferation of which causes secretion of an adhesive and protective matrix (biofilm), which further worsens nasal obstruction.

When nasal obstruction is not due to a deviation of the septum or is caused by nasal polyps, is currently treated by carrying out frequent nasal washing with physiological solutions, sea water, and/or thermal water to carry away mechanically from the nose the secretions that gather and to prevent and/or counter any possible infectious complications of bacterial origin. Washing in any case cannot act effectively on the congestion and inflammation of the mucosae, which at times aggravate the obstruction, and for this reason the patient tends to resort to, or even make undue use of, commonly used corticosteroid-based and sympathomimetic topical drugs for nasal decongestion, which, however, present the disadvantage of inducing adverse reactions at a local level, even causing atrophic rhinitis medicamentosa; said drugs are moreover not recommended for patients under the age of twelve on account of different reports of adverse reactions at a national and international level.

There would thus be necessary an intranasal mucosal composition that is able to moisten, decongest and, at the same time, prevent any possible bacterial complications that may be used in subjects under the age of twelve without giving rise to untoward reactions even with a prolonged and frequent use thereof over time.

It has now been surprisingly discovered that a composition for topical intranasal use containing carboxymethylßglucan and/or phosphoglucan with 18α and/or β glycyrrhetic acid and lactoferrin is able to moisten the mucosae disinflammating them and countering the aggregation and proliferation of the bacteria responsible for nasal obstruction.

The present invention consequently relates to a topical intranasal composition containing carboxymethylßglucan and/or phosphoglucan with 18α and/or β glycyrrhetic acid, possibly salified, and lactoferrin, useful in nasal obstruction.

It is known that carboxymethylßglucan and/or phosphoglucan are/is able to lubricate and immuno-balance the mucosae, improving the mucociliar mobility and reducing the hypertrophy of the turbinates (Passali D. , Fiorella R. , Campioni A. , Villari G. , Mora E. , Passali GC., Passali FM., Frisanti A., Bellussi L., Glucan solution spray vs saline in the treatment of chronic rhinosinusitis: a multi-centric double blind randomised clinical trial. Clin Ter. 2007 March-April; 158 (2): 139-45; EP 1 337 260.

As regards 18α and/or β glycyrrhetic acid, as likewise its most commonly used salts (potassium, ammonium), recent studies have proven that they exert an anti-inflammatory activity with sequestering action, binding to a pro-inflammatory protein referred to as HMGB1 (G. Faraco, S. Fossati, M. E. Bianchi, et al., (2007) "High mobility group box 1 protein is released by neural cells upon different stresses and worsens ischemic neurodegeneration in vitro and in vivo"-Journal of Neurochemistry, volume 103 (Issue 2), 590-603).

More in particular, the present applicant has surprisingly discovered that, amongst all the salts, the salts of silver and glucamine of 18α and/or β glycyrrhetic acid are particularly effective in preventing evolution of mucosal phlogosis correlated to nasal obstruction thanks to their capacity of preventing absorption of the acid.

In the composition according to the present invention there is moreover present lactoferrin, which has an antibacterial activity against the biofilm formed basically by Streptococcus, Stafilococcus, and Pseudomonas bacteria.

Lactoferrin is a natural endogenous chelating agent, involved in numerous biological mechanisms, which is normally found in human milk, cow's milk, saliva, tears, and bile, and chelates iron. It is a basic protein belonging to the family of "non-haem" ferritins, i. e., ferritins without the haem group (a polypeptide that chelates iron), which are able to bind iron with neutral or alkaline pH and release it with acidic pH.

Said proteins are synthetized by particular exocrine structures, such as mammary-gland cells, and other secretive. cells such as: tears, sweat, bile, seminal liquid, and pancreatic juice. It is accumulated in the granulocytes, and practically the glands of the entire class of mammals are able to produce this protein. Its function is precisely that of eliminating iron from the surrounding tissue where it is found. It acts by binding and absorbing iron, a substance that is fundamental for the nutriment of bacteria; the latter, if deprived of the cofactor, die. This mechanism is by now used or exploited in pharmacology in so far as bacteria, deprived of iron, are forced to abandon the colonies, which frequently tend to form, thus becoming more vulnerable to pharmacological treatment. In fact, biofilms have a bacterial mass such as to render ineffective even the most potent antibiotics: this fact has led to the development of a pharmacological technique in integrating or associating antimetabolites (antibiotics) to lactoferrins.

In the composition forming the subject of the present invention, there are moreover possibly present substances such as essential oils, for example mint, eucalyptol, cypress, lavander, and thyme as decongestants by virtue of the capacity to produce sensations of deobstruction.

All the aforementioned substances can be present in different weight ratios and more particularly as given hereinafter.

| **Components** | **Quantity** |
|---|---|
| D-Pantenol | 0.5 - 8 g |
| Lactoferrin | 0.1 - 3 g |
| Phosphoglucan and/or Carboxymethylbetaglucan | 1 - 50 g |
| Salt of 18 betaglycyrrhetic acid | 0.1 - 6 g |
| EDTA bisodium salt | 0.01 - 1 g |
| *N*-hydroxymethylglycinate sodium salt | 0.001 - 0.5 g |
| Chlorobutanol | 0.01 - 0.5 g |
| Natural eucalyptol aroma | 0.01 - 0.2 g |
| Cypress aroma | 0.01 - 0.2 g |
| Tween 20 | 0.05 - 5 g |
| Monobasic sodium phosphate | 0.01 - 0.5 g |
| Dibasic sodium phosphate | 0.01 - 1 g |
| Sodium hydroxide | 0.001 - 1 g |
| Demineralized water | up to 100 g |

The composition according to the present invention may obviously be prepared with techniques known in pharmaceutical technology and may be administered in the nasal cavities in the form of nasal spray or nasal drops and in any case in other known technical forms that are able to counter the effect of the symptoms due to nasal obstruction, such as, for example, washing and nasal ointments.

Another subject of the present invention regards the use of the topical nasal composition forming the subject of the invention for the treatment of nasal obstruction and in particular for the treatment of obstruction with bacterial complications.

Given hereinafter is a non-limiting example to provide a better illustration of the present invention.

### EXAMPLE

### Pharmaceutical form: Nasal spray

**Formula for 100 g:**

| **Components** | **Quantity** |
|---|---|
| D-Pantenol | 3.5 g |
| Lactoferrin | 0.5 g |
| Phosphoglucan | 20 g |
| Dipotassium glycyrrhizinate | 2 g |
| EDTA disodium salt | 0.1 g |
| Microglycin 50 | 0.1 g |
| Chlorobutanol | 0.2 g |
| Natural eucalyptol aroma | 0.02 g |
| Cypress aroma | 0.02 g |
| Tween 20 | 0.2 g |
| Monobasic sodium phosphate | 0.15 g |
| Dibasic sodium phosphate | 0.40 g |
| Sodium hydroxide | 0.096 g |
| Demineralized water | 72.714 g |

## Claims

1. A topical intranasal composition, **characterized in that** it contains carboxymethylßglucan and/or phosphoglucan with 18α and/or β glycyrrhetic acid, possibly salified, and lactoferrin for nasal obstruction.

2. The composition according to Claim 1, **characterized in that** for 100 g of composition, the carboxymethylßglucan and/or phosphoglucan is present in an amount of between 1-50 g, the salt of 18α and/or β glycyrrhetic acid is present in an amount of between 0.1 and 6 g, and the lactoferrin is present in an amount of between 0.1 and 3 g.

3. The composition according to either Claim 1 or Claim 2, **characterized in that** one or more essential oils are present, chosen between mint, eucalyptol, cypress, lavender, and thyme, each in an amount comprised between 0.01 and 0.2 g per 100 g of composition.

4. The composition according to any one of Claims 1 to 3, **characterized in that** the 18α and/or β glycyrrhetic acid is in the form of silver salt.

5. The composition according to any one of Claims 1 to 3, **characterized in that** the 18α and/or β glycyrrhetic acid is in the form of glycamin salt.

6. The composition according to any one of Claims 1 to. 5, **characterized in that** it comprises for 100 g:
| **Components** | **Quantity** |
|---|---|
| D-Pantenol | 0.5 - 8 g |
| Lactoferrin | 0.1 - 3 g |
| Phosphoglucan and/or Carboxymethylbetaglucan | 1 -,50 g |
| Salt of 18 betaglycyrrhetic acid | 0.1 - 6 g |
| EDTA bisodium salt | 0.01 - 1 g |
| N-hydroxymethylglycinate sodium salt | 0.001 - 0.5 g |
| Chlorobutanol | 0.01 - 0.5 g |
| Natural eucalyptol aroma | 0.01 - 0.2 g |
| Cypress aroma | 0.01 - 0.2 g |
| Tween 20 | 0.05 - 5 g |
| Monobasic sodium phosphate | 0.01 - 0.5 g |
| Dibasic sodium phosphate | 0.01 - 1 g |
| Sodium hydroxide | 0.001 - 1 g |
| Demineralized water | to 100 g |

7. The composition according to any one of Claims 1 to 6, **characterized in that** it is in the form of nasal spray.

8. The composition according to any one of Claims 1 to 5, **characterized in that** it is in the form of nasal drops.

9. Use of the composition according to any one of Claims 1 to 7 for the preparation of a topical intranasal medicament in the treatment of nasal obstruction, particularly in the treatment of nasal obstruction with bacterial complications.

## Patentansprüche

1. Topische intranasale Zusammensetzung, **dadurch gekennzeichnet, dass** sie Carboxymethyl-β-glucan und/oder Phosphoglucan mit 18α- und/oder β-Oycyrrhetinsäure, wahlweise in Salzform, und Lactoferrin zur Verhinderung der nasalen Verstopfung enthält.

2. Zusammensetzung nach Anspruch 1, **dadurch gekennzeichnet, dass** für 100 g der Zusammensetzung, das Carboxymethyl-β-glucan und/oder Phosphoglucan in einer Menge von zwischen 1 bis 50 g, das Salz von 18α- und/oder β-Glycyrrhetinsäure in einer Menge von zwischen 0,1 bis 6 g und das Lactoferrin in einer Menge von zwischen 0,1 bis 3 g vorliegt.

3. Zusammensetzung nach Anspruch 1 oder Anspruch 2, **dadurch gekennzeichnet, dass** ein oder mehrere essentielle Öle vorliegen, ausgewählt aus Minze, Eukalyptus, Zypresse, Lavendel und Thymian, jeweils in einer Menge von zwischen 0,01 bis 0,2 g pro 100 g der Zusammensetzung.

4. Zusammensetzung nach einem der Ansprüche 1 bis 3, **dadurch gekennzeichnet, dass** die 18α- und/oder β-Glycyrrhetinsäure in Form eines Silbersalzes vorliegt.

5. Zusammensetzung nach einem der Ansprüche 1 bis 3, **dadurch gekennzeichnet, dass** die 18α- und/oder β-Glycyrrhetinsäure in Form eines Glycaminsalzes vorliegt.

6. Zusammensetzung nach einem der Ansprüche 1 bis 5, **dadurch gekennzeichnet, dass** sie, bezogen auf 100 g, umfasst:
| **Verbindungen** | **Menge** |
|---|---|
| D-Pantenol | 0,5 - 8 g |
| Lactoferrin | 0,1 - 3 g |
| Phosphoglucan und/oder Carboxymethylbetaglucan | 1 - 50 g |
| Salz der 18β-Glycyrrhetinsäure | 0,1 - 6 g |
| EDTA-Binatriumsalz | 0,01 - 1 g |
| N-Hydroxymethylglycinat-Natriumsalz | 0,001 - 0,5 g |
| Chlorbutanol | 0,01 - 0,5 g |
| Natürliches Eukalyptusaroma | 0,01 - 0,2 g |
| Zypressenaroma | 0,01 - 0,2 g |
| Tween 20 | 0,05-5g |
| Monobasisches Natriumphosphat | 0,01 - 0,5 g |
| Dibasisches Natriumphosphat | 0,01-1g |
| Natriumhydroxid | 0,001 - 1 g |
| Demineralisiertes Wasser | Bis 100 g |

7. Zusammensetzung nach einem der Ansprüche 1 bis 6, **dadurch gekennzeichnet, dass** sie in Form eines Nasensprays vorliegt.

8. Zusammensetzung nach einem der Ansprüche 1 bis 5, **dadurch gekennzeichnet, dass** in Form von Nasentropfen vorliegt.

9. Verwendung der Zusammensetzung nach einem der Ansprüche 1 bis 7 zur Herstellung eines topischen intranasalen Medikaments zur Behandlung der Nasenverstopfung, insbesondere zur Behandlung der Nasenverstopfung mit bakteriellen Komplikationen.

## Revendications

1. Composition intra-nasale topique, **caractérisée en ce qu'**elle contient du carboxyméthyl-β-glucane et/ou du phosphoglucane avec de l'acide 180α et/ou β-glycyrrhétinique, éventuellement salifié, et de la lactoferrine, pour obstruction nasale.

2. Composition selon la revendication 1, **caractérisée en ce que** pour 100 g de composition, le carboxy-méthyl-β-glucane et/ou le phosphoglucane est présent en une quantité entre 1 et 50 g, le sel d'acide 18α et/ou β-glycyrrhétinique est présent en une quantité entre 0,1 et 6 g et la lactoferrine est présente en une quantité entre 0,1 et 3 g.

3. Composition selon la revendication 1 ou la revendication 2, **caractérisée en ce qu'**une ou plusieurs huiles essentielles sont présentes, choisies parmi la menthe, l'eucalyptol, le cyprès, la lavande et le thym, chacune en une quantité comprise entre 0,01 et 0,2 g pour 100 g de composition.

4. Composition selon l'une quelconque des revendications 1 à 3, **caractérisée en ce que** l'acide 18α et/ou β glycyrrhétinique se présente sous la forme de sel d'argent.

5. Composition selon l'une quelconque des revendications 1 à 3, **caractérisée en ce que** l'acide 18α et/ou β glycyrrhétinique se présente sous la forme de sel de glycamine.

6. Composition selon l'une quelconque des revendications 1 à 5, **caractérisée en ce qu'**elle comprend pour 100 g :
| COMPOSANTS | QUANTITÉ |
|---|---|
| D-panténol | 0,5 à 8 g |
| Lactoferrine | 0,1 à 3 g |
| Phosphoglucane et/ou Carboxyméthylbêtaglucane | 1 à 50 g |
| Sel d'acide 18 bêta-glycyrrhétinique | 0,1 à 6 g |
| Sel bisodique EDTA | 0,01 à 1 g |
| Sel de N-hydroxyméthylglycinate sodium | 0,001 à 0,5 g |
| Chlorobutanol | 0,01 à 0,5 g |
| Arôme d'eucalyptol naturel | 0,01 à 0, 2 g |
| Arôme de cyprès | 0,01 à 0, 2 g |
| Tween 20 | 0,05 à 5 g |
| Phosphate de sodium monobasique | 0,01 à 0,5 g |
| Phosphate de sodium dibasique | 0,01 à 1 g |
| Hydroxyde de sodium | 0,001 à 1 g |
| Eau déminéralisée | Jusqu'à 100 g |

7. Composition selon l'une quelconque des revendications 1 à 6, **caractérisée en ce qu'**elle se présente sous la forme d'un spray nasal.

8. Composition selon l'une quelconque des revendications 1 à 5, **caractérisée en ce qu'**elle se présente sous la forme de gouttes nasales.

9. Utilisation de la composition selon l'une quelconque des revendications 1 à 7, pour la préparation d'un médicament intra-nasal topique dans le traitement de l'obstruction nasale, en particulier dans le traitement de l'obstruction nasale avec complications bactériennes.
